# EUROPÄISCHE PATENTSCHRIFT

(11) **EP 1 974 658 B1**
(45) Veröffentlichungstag und Bekanntmachung des Hinweises auf die Patenterteilung: **25.11.2009**
(21) Anmeldenummer: 07006359.9
(22) Anmeldetag: 28.03.2007
(51) Int. Cl.: A61B 5/00

(54) **Schlossmechanismus zum Verriegeln eines Analysegerätes in der Produktion**
Locking mechanism for locking an analysis device in the production area
Mécanisme de serrure destiné au verrouillage d'un appareil d'analyse en production

(43) Veröffentlichungstag der Anmeldung: 01.10.2008
(73) Patentinhaber: Roche Diagnostics GmbH, 68305 Mannheim (DE); F.HOFFMANN-LA ROCHE AG, 4070 Basel (CH)
(72) Erfinder: Meinecke, Dieter, 68309 Mannheim (DE); Riebel, Stefan, 6330 Cham (CH); Pelzer, Wolfgang, 52372 Kreuzau (DE); Reubzaet, Wouter, 6444 KN Brunssum (NL)
(74) Vertreter: Jany, Peter

(56) Entgegenhaltungen:
- WO-A-2005/054811
- US-A- 3 668 476
- US-A1- 2004 138 588
- US-A1- 2006 030 194

## Beschreibung

Die Erfindung betrifft ein Analysegerät zum Analysieren eines medizinisch bedeutsamen Bestandteils einer Körperflüssigkeit, beispielsweise Blut oder interstitielle Flüssigkeit. Dabei wird eine qualitative oder quantitative Analyse durchgeführt, also beispielsweise das Vorhandensein, das Nichtvorhandensein oder die Konzentration eines bestimmten Analyten in einer Probe bestimmt. Die Erfindung bezieht sich insbesondere auf ein tragbares, von einem Patienten bedienbares Analysegerät für die Patienten-Selbstkontrolle, den sogenannten Bereich des Home Monitoring, insbesondere ein Blutglukosemessgerät, ein Cholesterin-Messgerät oder ein Blutgerinnungsparameter-Messgerät

Derartige Analysegeräte umfassen ein Gerätegehäuse, eine in dem Gerätegehäuse angeordnete Messeinrichtung zum Durchführen der Analyse an einer Probe und einen Prozessor mit Software zum Verarbeiten der von der Messeinrichtung ermittelten Messwerte und zum Aufbereiten der Analysemessdaten aus den Messwerten, in der Regel unter Berücksichtigung von Kalibrationswerten. Die Probe kann beispielsweise auf einem Testelement durch eine Gehäuseöffnung in das Analysegerät und in die Messeinrichtung eingeführt werden. In anderen Ausführungsformen werden auch Analysegeräte verwendet, bei denen die Probe bzw. die mit einer Probe benetzten Testelemente, beispielsweise auf einem Band, an einem Mess-Sensor des Analysegerätes vorbeigeführt werden, der sich an der Oberfläche des Analysegerätes befindet oder aus diesem herausragt. Auch die Verwendung von Magazinen für Testelemente ist in diesem Zusammenhang bekannt.

Zur qualitativen und quantitativen Analyse von Bestandteilen einer flüssigen Probe, insbesondere einer Körperflüssigkeit von Menschen oder Tieren, werden in großem Umfang Testverfahren eingesetzt, die mit Testelementen arbeiten. Die Testelemente enthalten Reagenzien. Zur Durchführung einer Reaktion wird das Testelement mit der Probe in Kontakt gebracht. Die Reaktion von Probe und Reagenz führt zu einer für die Analyse charakteristischen Veränderung des Testelements, die mit Hilfe eines geeigneten Analysegerätes ausgewertet wird. Das Analysegerät ist in der Regel zum Auswerten eines ganz bestimmten Typs von Testelementen eines bestimmten Herstellers geeignet. Die Testelemente und das Analysegerät bilden wechselseitig aufeinander abgestimmte Bestandteile und werden insgesamt als Analysesystem bezeichnet.

Es sind zahlreiche unterschiedliche Testelement-Typen bekannt, die sich durch das Messprinzip und die verwendeten Reagenzien sowie durch ihren Aufbau unterscheiden.

Hinsichtlich des Messprinzips sind kolorimetrische Analysesysteme besonders weit verbreitet. Bei ihnen führt die Reaktion der Probe mit den in dem Testelement enthaltenen Reagenzien zu einer Farbänderung, die visuell oder mittels einer photometrischen Messreinrichtung gemessen werden kann. Daneben haben elektrochemische Analysesysteme eine große Bedeutung erlangt, bei denen die Reaktion der Probe mit den Reagenzien des Testelements zu einer elektrisch messbaren Änderung (einer elektrischen Spannung oder eines elektrischen Stromes) führt, die mit einer entsprechenden Messelektronik gemessen wird. Derartige Analysesysteme werden auch als amperometrische Systeme bezeichnet.

Häufig ist eine regelmäßige Überwachung bestimmter analytischer Werte des Blutes erforderlich. Dies gilt insbesondere für Diabetiker, die mittels einer Blutzucker-Selbstkontrolle ihren Blutzuckerspiegel häufig kontrollieren sollten, um durch Anpassung von Insulininjektionen an den stark schwankenden Bedarf ihren Blutzuckerspiegel möglichst ständig innerhalb bestimmter Sollgrenzen zu halten. Entsprechend ist auch die Überprüfung von Blutgerinnungsparametern durch eine Patienten-Blutgerinnungs-Selbstkontrolle verbreitet.

Ein Blutglukosemessgerät ist ein Messgerät, mit dessen Hilfe der Blutzuckergehalt qualitativ oder quantitativ bestimmt werden kann. In der Regel wird hierzu in einem Körper eine Einstichwunde erzeugt, ein Bluttropfen entnommen, der Bluttropfen auf das Testelement aufgebracht und mit Hilfe des Testelements und des Blutglukosemessgeräts der Blutglukosegehalt in dem Tropfen bestimmt. Es ist aber auch denkbar, durch eine permanente Messung, beispielsweise mit in den Körper eingeführten Sensoren oder durch die Haut hindurch die Blutglukose zu messen.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglukosekonzentration, ist es wichtig, dass eine einfache und zuverlässige Bedienung des Blutglukosemessgeräts möglich sowie eine informative und verlässliche Bestimmung und Darstellung der Messergebnisse vorhanden ist.

Die gebräuchlichen Analysegeräte sind sogenannte Stand-Alone-Messgeräte. Diese Geräte arbeiten autonom, selbständig und unabhängig. Sie haben also ein Display, eine Messeinrichtung, eine Stromversorgung und ein vollständiges User-Interface, das z.B. eine Tastatur, eine Anzeige, einen Signalgeber oder eine Benutzerführung umfassen kann. Der Anwendungszweck und die Eigenschaften derartiger Geräte sind, bis auf gelegentliche Anpassungen der Firmware, festgelegt (z.B. US 2004/0138588, WO 2005/054811).

Die Erfindung bezieht sich insbesondere auf die Fertigung derartiger Analysegeräte im Werk, beispielsweise die Montage von Gehäuseteilen, wie sie z.B. für Blutzuckermessgeräte verwendet werden. Hierbei umfasst das Gehäuse beispielsweise zwei Teile, nämlich eine Gehäuseoberschale und eine Gehäuseunterschale, wobei das Gehäuse in der Gehäuseoberfläche Bedienelemente, z.B. eine Tastatur, für das Blutzuckermessgerät umfasst. Eine Funktionskontrolle des Messgerätes erfolgt, nachdem die beiden Gehäusehälften zusammengefügt wurden, zum Abschluss der Montage des Analysegerätes. Nach dem Stand der Technik werden häufig Schnappverbindungen eingesetzt, um die Montage möglichst günstig zu gestalten. Ein erneutes Auseinandernehmen der einmal zusammengefügten Gehäusehälften ist dann nicht möglich, da die Montage der Anforderung genügen muss, ein durch den Endbenutzer nicht zu öffnendes Gehäuse zu gewährleisten. Sofern daher bei der Endkontrolle der Gerätefunktionen Fehler festgestellt werden, z.B. bei den Bedienelementen, müssen folglich zur Reparatur des Gerätes die Gehäusehälften beim Öffnen zerstört werden.

Bisher werden Analysemessgeräte im letzten Montageschritt entweder durch eine Verschraubung miteinander verbunden oder es werden die Gehäuseteile miteinander verrastet. Da es wichtig ist, dass man erkennen kann ob ein Gerät, das vom Kunden wegen einer Fehlfunktion an den Hersteller zur Reparatur zurückgesandt wird, bereits geöffnet wurde, ist die Verrastung dabei so ausgelegt, dass sie beim Wideröffnen des Gerätes zerstört wird. Schrauben werden normalerweise durch ein Siegel, ein Label oder Schutzlack abgedeckt. In diesem Fall muss zum Öffnen des Gerätes das Label abgezogen werden (was zur Beschädigung des Labels führt) oder es wird der Schutzlack auf der Schraube beschädigt.

Versiegelungen und Verplombungen von Behältern oder Geräten, die das Öffnen verhindern oder eine nur einmalige Benutzung des Gerätes ermöglichen, sind beispielsweise aus folgenden Druckschriften bekannt: US 3,753,586, US 4,834,706, US 4,875,486, US 1,487,885, DE 4240327 C2, GB 984,593, GB 850,385, GB 2 040 267 A, US 6,685,085 B2, WO 98/19723, US 2005/0227370 A1, US 4,663,970, DE 27 53 285 A1, US 2,772,109, US 4,416,478, US 2,142,048, FR 970,463, US 2,081,627, US 1,995,878, GB 1 475 543, US 3, 668, 476, US 2006/0030194.

Nach der Montage eines Analysegerätes, beispielsweise bei der Herstellung oder nach Durchführung einer Reparatur oder Wartung, wird eine finale Funktionsprüfung durchgeführt. Hierfür muss nach dem Stand der Technik das Gerät komplett montiert sein. Tritt an dieser Stelle ein Fehler auf, so muss das Gerät wieder demontiert und repariert werden. Der Nachteil der Verschraubung bzw. der nicht lösbaren Verrastung besteht hier darin, dass nach der Demontage Bauteile nicht wieder verwendet werden können und es somit zu erhöhten Kosten kommt. Bei einer Verrastung sind hierbei vor allem die Bauteile mit den Rasthaken betroffen. Bei verschraubten Teilen ist die Wiederverwendung der Bauteile mit den Schraubdomen kritisch. Zusätzlich erhöht der Einsatz von Schraubverbindungen den Montageaufwand und somit die Kosten.

Der Erfindung liegt die Aufgabe zugrunde, ein Analysegerät zu schaffen, dessen Gehäuse bei der Montage erst nach Durchführung der Funktionskontrolle derart verschlossen werden kann, dass es gegen ein unerwünschtes Öffnen gesichert bzw. ein solches Öffnen an dem Gerät erkennbar ist, wobei vorzugsweise bei dem dabei verwendeten Schlosselement nicht vom Benutzer des Gerätes die Schlossfunktion erkennbar sein soll. Ferner richtet sich die Erfindung auf ein entsprechendes Verfahren zum Herstellen eines Analysegerätes mit einer verbesserten Möglichkeit der Funktionsprüfung in der Fertigung.

Diese Aufgabe wird erfindungsgemäß durch ein Analysegerät bzw. ein Verfahren mit den Merkmalen der beigefügten unabhängigen Patentansprüche gelöst. Bevorzugte Ausgestaltungen und Weiterbildungen der Erfindung ergeben sich aus den abhängigen Patentansprüchen und der nachfolgenden Beschreibung mit zugehöriger Zeichnung.

Ein erfindungsgemäßes Analysegerät zum Analysieren eines medizinisch bedeutsamen Bestandteils einer Probe, insbesondere ein Blutglukosemessgerät, umfassend ein Gerätegehäuse mit mindestens zwei Gehäuseteilen, eine in dem Gerätegehäuse angeordnete Messeinrichtung zum Durchführen der Analyse an einer Probe, und einen Prozessor mit Software zum Verarbeiten der von der Messeinrichtung ermittelten Messwerte und zum Aufbereiten von Analysemessdaten aus den Messwerten, weist also die Besonderheit auf, dass das Analysegerät ein Schlosselement umfasst, das funktional der Verriegelung des Analysegerätes durch Herstellung einer festen mechanischen Verbindung zwischen den mindestens zwei Gehäuseteilen dient, wobei das Analysegerät und das Schlosselement derart ausgebildet sind, dass das Analysegerät auch ohne das Schlosselement funktionsfähig ist, derart gestaltet ist, dass bei der Montage des Analysegerätes zur Durchführung einer funktionalen Endkontrolle des, bis auf das Schlosselement fertig montierten Analysegerätes, die Funktion des Analysegerätes geprüft und erforderlichenfalls das Gehäuse zur Instandsetzung zerstörungsfrei geöffnet werden kann, und derart gestaltet ist, dass bei der der Montage des Schlosselements an dem Gehäuse, insbesondere nach Durchführung einer funktionalen Endkontrolle des Analysegerätes, das Gehäuse verriegelt wird, so dass danach ein Wiederöffnen des Gehäuses nicht mehr möglich ist, ohne dass dieses Wiederöffnen an dem Gehäuse oder dem Schlosselement erkennbar ist.

Das Schlosselement hat vorzugsweise keine Funktion für die Bedienung und für Bedienelemente des Analysegerätes bzw. keine Funktion für das Funktionieren oder vollständige Funktionieren des Analysegerätes. Dabei kann es ausschließlich funktional der Verriegelung des Analysegerätes durch Herstellung einer festen mechanischen Verbindung zwischen den mindestens zwei Gehäuseteilen dienen oder ggf. in anderer Hinsicht, insbesondere in Bezug auf die Formgebung und/oder Handhabung des Analysegerätes funktionell zweckmäßig ausgebildet sein. Bevorzugt ist das Schlosselement derart ausgebildet, dass es keine Funktion ausübt, die für das vollständige Funktionieren des Analysegerätes erforderlich ist; dieses Merkmal ist so zu verstehen, dass das Analysegerät auch ohne Schlosselement funktionstüchtig oder komplett funktionstüchtig ist, was nicht ausschließt, dass von dem Schlosselement Zusatzfunktionen ausgehen, beispielsweise hinsichtlich des Designs, der manuellen Handhabung, beispielsweise das Bereitstellen von Greifelementen zum Ergreifen des Analysegerätes, oder sonstige in das Schlosselement integrierte oder von dem Schlosselement alleine oder im Zusammenwirken mit dem Analysegerät für das Analysegerät bereitgestellte Zusatzfunktionen.

Erfindungsgemäß kann ein solches Schlosselement bei der Montage des Analysegerätes verwendet werden, so dass die zwei oder mehr Gehäusehälften zerstörungsfrei wieder geöffnet werden können. Erst nach Montage des Schlosselementes ist ein Wiederöffnen des Gehäuses nicht mehr möglich. Die erfindungsgemäße Lösung besteht also darin, dass ein zusätzliches Gehäuseteil einsetzt wird, das funktional (ausschließlich) der Verriegelung des Analysegerätes dient. Anders als z.B. bei Plomben kann dieses Bauteil jedoch ein Bestandteil des Gesamtgehäuses sein und sich in das Design einfügen, so dass das der Verriegelung des Gehäuses dienende "Schloss" nicht als solches für den Benutzer des Analysegerätes zu erkennen ist.

Da das Schlosselement keine Funktion ausübt, die für das (vollständige) Funktionieren des Analysegerätes erforderlich ist, kann nun bei der Montage das Gerät bis auf das Schloss komplett aufgebaut und geprüft werden. Tritt ein Fehler auf, so kann eine beschädigungsfreie Demontage und Reparatur erfolgen. Erst nach erfolgreicher Prüfung wird das Schloss montiert und somit das Gerät verriegelt. Die Verriegelung ist dabei vorzugsweise so ausgelegt, dass es beim Öffnen zu einer Beschädigung des Schlossbauteils und/oder des Gehäuses kommt und somit ist die Erkennbarkeit des Öffnens durch den Anwender gewährleistet.

Ein erfindungsgemäßes Verfahren zum Herstellen eines Analysegerätes zum Analysieren eines medizinisch bedeutsamen Bestandteils einer Probe, insbesondere eines Blutglukosemessgerätes, wobei das Analysegerät ein Gerätegehäuse mit mindestens zwei Gehäuseteilen, eine in dem Gerätegehäuse angeordnete Messeinrichtung zum Durchführen der Analyse an einer Probe, und einen Prozessor mit Software zum Verarbeiten der von der Messeinrichtung ermittelten Messwerte und zum Aufbereiten von Analysemessdaten aus den Messwerten umfasst, und wobei das Analysegerät bei seiner Herstellung einer Funktionskontrolle unterzogen und mit einem Schlosselement geschlossen wird, weist dementsprechend die Besonderheit auf, dass das Analysegerät mit einem Schlosselement geschlossen wird, das funktional der Verriegelung des Analysegerätes durch Herstellung einer festen mechanischen Verbindung zwischen den mindestens zwei Gehäuseteilen dient, wobei das Analysegerät und das Schlosselement derart ausgebildet sind, dass das Analysegerät auch ohne das Schlosselement funktionsfähig ist, derart gestaltet ist, dass bei der Montage des Analysegerätes zur Durchführung einer funktionalen Endkontrolle des, bis auf das Schlosselement fertig montierten Analysegerätes, die Funktion des Analysegerätes geprüft und erforderlichenfalls das Gehäuse zur Instandsetzung zerstörungsfrei geöffnet werden kann, und derart gestaltet ist, dass bei der der Montage des Schlosselements an dem Gehäuse, insbesondere nach Durchführung einer funktionalen Endkontrolle des Analysegerätes, das Gehäuse verriegelt wird, so dass danach ein Wiederöffnen des Gehäuses nicht mehr möglich ist, ohne dass dieses Wiederöffnen an dem Gehäuse oder dem Schlosselement erkennbar ist, und dass bei der Montage des Analysegerätes zur Durchführung einer funktionalen Endkontrolle des, bis auf das Schlosselement fertig montierten Analysegerätes, die Funktion des Analysegerätes geprüft und danach bei der der Montage des Schlosselements an dem Gehäuse, nach Durchführung einer funktionalen Endkontrolle des Analysegerätes, das Gehäuse verriegelt wird.

Dabei kann in vorteilhafter Ausgestaltung vorgesehen sein, dass bei der Montage des Analysegerätes nach Durchführung einer funktionalen Endkontrolle des, bis auf das Schlosselement fertig montierten Analysegerätes, die Funktion des Analysegerätes geprüft, im Falle eines festgestellten Funktionsfehlers das Gehäuse zur Fehlerbehebung oder Instandsetzung zerstörungsfrei geöffnet und der Fehler behoben wird, anschließend zur Durchführung einer funktionalen Endkontrolle des, bis auf das Schlosselement fertig montierten Analysegerätes, die Funktion des Analysegerätes erneut geprüft und danach bei der der Montage des Schlosselements an dem Gehäuse, nach Durchführung der erneuten funktionalen Endkontrolle des Analysegerätes, das Gehäuse verriegelt wird.

Weitere Einzelheiten und Vorteile der Erfindung werden anhand eines Ausführungsbeispiels unter Bezugnahme auf die beigefügte Zeichnung erläutert. Die beschriebenen Merkmale können einzeln oder in Kombination verwendet werden, um bevorzugte Ausgestaltungen der Erfindung zu schaffen.

Es zeigen:
Fig. 1 ein erfindungsgemäßes Analysegerät, das zur Durchführung einer Funktionsendkontrolle fertig montiert ist, aber noch ohne erfindungsgemäßes Schlosselement;
Fig. 2 das Anbringen eines erfindungsgemäßen Schlosselements an das fertig geprüfte Analysegerät von Fig. 1;
Fig. 3 eine andere Ansicht zu Fig. 2; und
Fig. 4 das fertig montierte Analysegerät von Fig. 1 bis 3 mit eingerastetem erfindungsgemäßem Schlosselement.

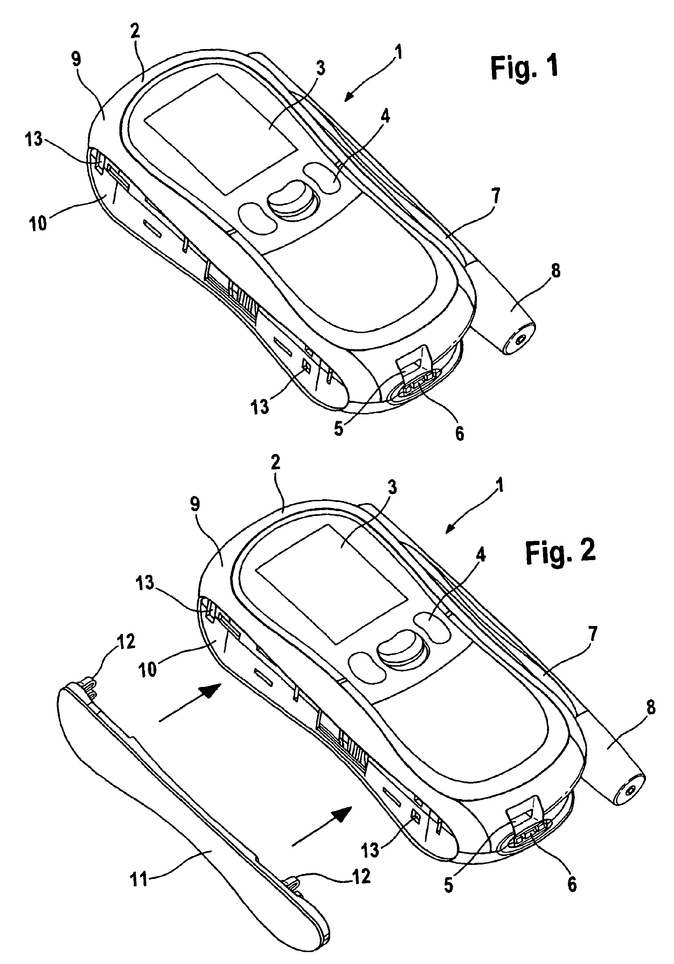

Die Fig. 1 zeigt ein erfindungsgemäßes Analysegerät. Bei einem solchen Analysegerät 1 handelt es sich beispielsweise um ein Analysegerät für die Analyse einer Körperflüssigkeit, insbesondere für die Patienten-Selbstkontrolle, beispielsweise um ein Blutglukosemeter, ein Cholesterin-Messgerät oder ein Blutgerinnungsparameter-Messgerät. Bei dem in Fig. 1 dargestellten Analysegerät 1 handelt es sich um ein tragbares, von einem Patienten manuell handhabbares, manuell betätigbares und manuell bedienbares Analysegerät in Form eines Blutglukosemessgerätes für die Patienten-Selbstkontrolle von Blutglukosewerten. Es umfasst ein Gehäuse 2 mit einer Anzeige 3, Bedienelementen 4 und einer Öffnung 5 zum Einführen eines Testelements mit einer Probe, die mit dem Analysegerät 1 analysiert wird.

In dem Gehäuse 2 des Blutglukosemessgeräts ist eine Messeinrichtung zum Durchführen einer Blutglukosebestimmung angeordnet. Die Blutglukosewerte werden mittels eines von dem Patienten gewonnenen Bluttropfens durchgeführt, der auf ein Testfeld eines Testelements aufgebracht wird. Das Testelement kann durch die Öffnung 5 in dem Gehäuse 2 in das Blutglukosemessgerät und in die darin befindliche Messeinrichtung eingeführt werden. Eine solche Messeinrichtung kann beispielsweise eine kolorimetrische oder elektrochemische Messeinrichtung sein. In anderen Ausführungsformen befinden sich die Testelemente in dem Gehäuse 2, beispielsweise in Form von Magazinen, und werden durch eine Öffnung in dem Gehäuse 2 mit dem Bluttropfen benetzt.

Bei dem Blutglukosemessgerät kann es sich alternativ auch um ein integriertes Gerät mit Streifenmagazin handeln. Hierunter ist ein Gerät zu verstehen, bei dem sogenannte integrierte Disposables verwendet werden. Integrierte Disposables sind Verbrauchselemente, die sich dadurch auszeichnen, das sie sowohl ein Nadelelement zum Durchführen des Einstichvorgangs für das Gewinnen einer Körperflüssigkeitsprobe, beispielsweise einer Blutprobe, als auch eine Testchemie zum Durchführen der Analyse eines medizinisch bedeutsamen Bestandteils der Probe beinhalten. Solche Disposables werden häufig in geeigneten Magazinen, die z.B. die Form eines Streifens haben, verwendet.

Das Blutglukosemessgerät führt, ggf. unter Berücksichtigung von Kalibrationswerten, mittels eines Prozessors die Blutglukosebestimmung durch und überträgt die ermittelten Analysemessdaten an die integrierte Anzeige 3 und/oder über eine Schnittstelle 6 an eine externe Anzeige oder einen Computer.

Das Analysegerät 1 von Fig. 1 umfasst noch einen Halter 7 mit einer abnehmbaren Stecheinrichtung 8 zum Gewinnen des Bluttropfens für die Durchführung der Analyse.

Das Gehäuse 2 des Analysegeräts 1 umfasst ein oberes Gehäuseteil 9 und ein unteres Gehäuseteil 10, die ebenso wie der Rest des Analysegerätes 1, ausgenommen das auf der linken Gehäuseseite noch anzufügende Schlosselement, zum Durchführen einer finalen Funktionsprüfung montiert sind. Da das Analysegerät 1 fertig montiert ist, ausgenommen das Schlosselement, kann an ihm eine vollständige Funktionskontrolle durchgeführt werden, und, falls eine Reparatur erforderlich sein sollte, kann es ohne Probleme und ohne Zerstörung von Bauteilen geöffnet werden.

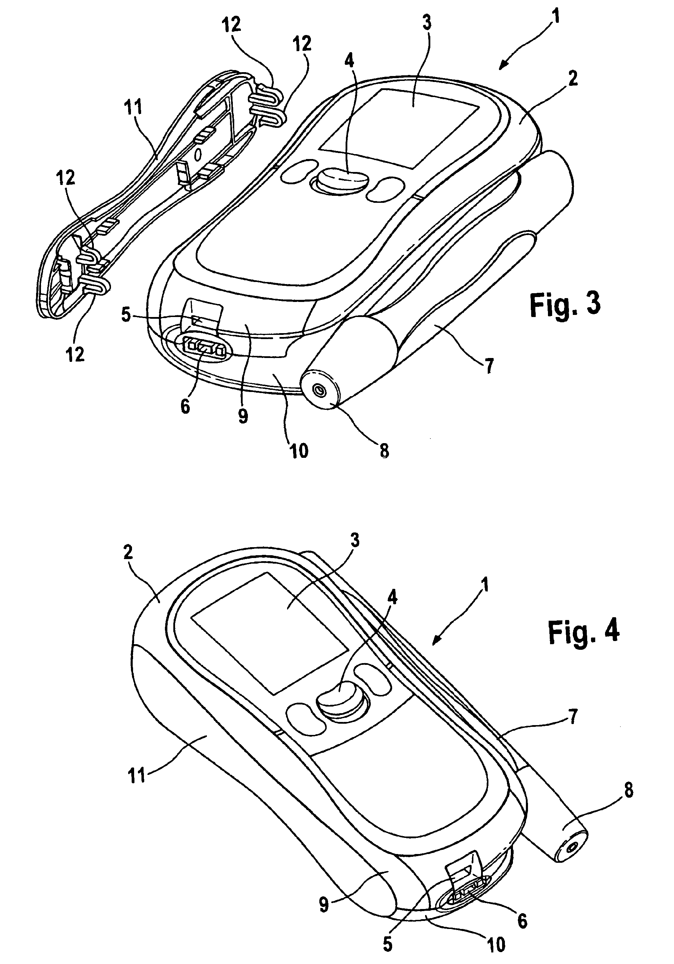

Die Fig. 2 zeigt das Anbringen eines erfindungsgemäßen Schlosselements 11 an das Gehäuse 1 des fertig geprüften Analysegeräts 1 von Fig. 1. Nach erfolgreich bestandener Prüfung kann das Schlosselement 11 aufgebracht werden, wobei das Schlosselement derart in Gehäuseteile des Gehäuses 1 einrastet, dass eine zerstörungsfreie Demontage des Schlosselements 11 nicht mehr möglich ist. Hierzu weist das Schlosselement 11 Rasthaken 12 auf, die zum Einführen in korrespondierende Aufnahmeöffnungen 13 des Gehäuses ausgebildet sind. Die Fig. 3 zeigt eine andere Ansicht zu Fig. 2. Man erkennt, dass in diesem Ausführungsbeispiel das Schlosselement 11 insgesamt vier Rasthaken 12 aufweist, nämlich jeweils zwei an einem Ende des Schlosselements 11, wobei diese zwei Rasthaken jeweils nebeneinander angeordnet sind und jeweils einer dieser zwei Rasthaken in das obere Gehäuseteil 9 und der andere, benachbarte Rasthaken 12 in das untere Gehäuseteil 12 einrastet. Diese Rasthaken 12 stellen Sollbruchstellen dar, die beim Öffnen des Analysegerätes 1 bzw. beim Abnehmen des Schlosselements 11 von dem Gehäuse 2 brechen. Auf diese Weise kann ein nachträgliches oder unautorisiertes Öffnen des Analysegerätes 1 erkannt werden.

In anderen Ausführungsformen ist es auch möglich, dass das Schlosselement 11 keine Sollbruchstelle aufweist und derart mit dem Gehäuse 2 verbunden wird, beispielsweise durch Rasthaken 12, dass es beim Öffnen des Analysegerätes 1 bzw. beim Abnehmen des Schlosselements 11 zerstört wird. Allgemein ist es vorteilhaft, wenn die Verriegelung des Gehäuses 2 durch das Schlosselement 11 derart ausgebildet ist, dass es beim Öffnen des Gehäuses 1 zu einer Beschädigung des Schlosselements 11 kommt, durch welche die Erkennbarkeit des Öffnens des Gehäuses 2 gewährleistet ist.

Die Fig. 4 zeigt das fertig montierte Analysegerät 1 von Fig. 1 bis 3 mit eingerastetem erfindungsgemäßem Schlosselement 11. Man erkennt, dass das Schlosselement 11 als zusätzliches Gehäuseteil ausgebildet ist, das mindestens zwei Gehäuseteile, nämlich das obere Gehäuseteil 9 und das untere Gehäuseteil 10 verbindet, und sich dabei derart in das Design des Gehäuses 2 einfügt, dass seine Funktion als Schloss als solche nicht für den Benutzer des Analysegerätes 1 erkennbar ist. Das Schlosselement 11 ist ein Bestandteil des Gesamtgehäuses, und es kann als Gehäuseteil ausgebildet oder in ein Gehäuseteil integriert sein.

Eine bevorzugte Ausbildung besteht darin, dass das Schlosselement 11 tragender Bestandteil der Gehäusekonstruktion ist, so dass es zu dem mechanischen Zusammenhalt der Gehäuseteile beiträgt. Bei dem dargestellten Ausführungsbeispiel ist das Schlosselement 11 ein Hartplastikteil, das an der linken Seite des Analysegerätes 1 platziert ist, an dieser Stelle den seitlichen Abschluss des Gehäuses 2 bildet und durch seine Formgebung als Griffstück zum sicheren Greifen des Analysegerätes 1 durch den Anwender ausgebildet ist.

### Bezugszeichenliste

- 1: Analysegerät
- 2: Gehäuse
- 3: Anzeige
- 4: Bedienelemente
- 5: Öffnung
- 6: Schnittstelle
- 7: Halter
- 8: Stecheinrichtung
- 9: Oberes Gehäuseteil
- 10: Unteres Gehäuseteil
- 11: Schlosselement
- 12: Rasthaken
- 13: Aufnahmeöffnung

## Patentansprüche

1. Analysegerät (1) zum Analysieren eines medizinisch bedeutsamen Bestandteils einer Probe, insbesondere ein Blutglukosemessgerät, umfassend
ein Gerätegehäuse (2) mit mindestens zwei Gehäuseteilen (9, 10),
eine in dem Gerätegehäuse (2) angeordnete Messeinrichtung zum Durchführen der Analyse an einer Probe, und
einen Prozessor mit Software zum Verarbeiten der von der Messeinrichtung ermittelten Messwerte und zum Aufbereiten von Analysemessdaten aus den Messwerten,
**dadurch gekennzeichnet, dass**
das Analysegerät (1) ein Schlosselement (11) umfasst, das
funktional der Verriegelung des Analysegerätes (1) durch Herstellung einer festen mechanischen Verbindung zwischen den mindestens zwei Gehäuseteilen (8, 9) dient, wobei das Analysegerät (1) und das Schlosselement (11) derart ausgebildet sind, dass das Analysegerät (1) auch ohne das Schlosselement (11) funktionsfähig ist,
derart gestaltet ist, dass bei der Montage des Analysegerätes (1) zur Durchführung einer funktionalen Endkontrolle des, bis auf das Schlosselement (11) fertig montierten Analysegerätes (1), die Funktion des Analysegerätes (1) geprüft und erforderlichenfalls das Gehäuse (2) zur Instandsetzung zerstörungsfrei geöffnet werden kann, und
derart gestaltet ist, dass bei der Montage des Schlosselements (11) an dem Gehäuse (2), insbesondere nach Durchführung einer funktionalen Endkontrolle des Analysegerätes (1), das Gehäuse (2) verriegelt wird, so dass danach ein Wiederöffnen des Gehäuses (2) nicht mehr möglich ist, ohne dass dieses Wiederöffnen an dem Gehäuse (2) oder dem Schlosselement (11) erkennbar ist,
und dass das Schlosselement (11) als zusätzliches Gehäuseteil ausgebildet ist, das mindestens zwei Gehäuseteile (9, 10) verbindet.

2. Analysegerät (1) nach Anspruch 1, **dadurch gekennzeichnet, dass** die Verriegelung des Gehäuses (2) durch das Schlosselement (11) derart ausgebildet ist, dass es beim Öffnen des Gehäuses (2) zu einer Beschädigung des Schlosselements (11) kommt, durch welche die Erkennbarkeit des Öffnens des Gehäuses (2) gewährleistet ist.

3. Analysegerät (1) nach einem der vorhergehenden Ansprüche, **dadurch gekennzeichnet, dass** das Schlosselement (11) derart in Gehäuseteile (9, 10) einrastet, dass eine zerstörungsfreie Demontage des Schlosselements (11) nicht mehr möglich ist.

4. Analysegerät (1) nach einem der vorhergehenden Ansprüche, **dadurch gekennzeichnet, dass** das Schlosselement (11) Rasthaken (12) aufweist, die zum Einführen in korrespondierende Aufnahmeöffnungen (13) des Gehäuses (2) ausgebildet sind.

5. Analysegerät (1) nach einem der vorhergehenden Ansprüche, **dadurch gekennzeichnet, dass** das Schlosselement (11) eine Sollbruchstelle aufweist, die beim Öffnen des Analysegerätes (1) bzw. Abnehmen des Schlosselements (11) bricht.

6. Analysegerät (1) nach einem der vorhergehenden Ansprüche, **dadurch gekennzeichnet, dass** das Schlosselement (11) keine Sollbruchstelle aufweist, so das es beim Öffnen des Analysegerätes (1) bzw. Abnehmen des Schlosselements (11) zerstört wird.

7. Analysegerät (1) nach einem der vorhergehenden Ansprüche, **dadurch gekennzeichnet, dass** das Schlosselement (11) ein Bestandteil des Gesamtgehäuses ist.

8. Analysegerät (1) nach einem der vorhergehenden Ansprüche, **dadurch gekennzeichnet, dass** das Schlosselement (11) als Gehäuseteil ausgebildet oder in ein Gehäuseteil integriert ist.

9. Analysegerät (1) nach einem der vorhergehenden Ansprüche, **dadurch gekennzeichnet, dass** das Schlosselement (11) tragender Bestandteil der Gehäusekonstruktion ist, so dass es zu dem mechanischen Zusammenhalt der Gehäuseteile (9, 10) beiträgt.

10. Analysegerät (1) nach einem der vorhergehenden Ansprüche, **dadurch gekennzeichnet, dass** sich das Schlosselement (11) derart in das Design des Gehäuses (2) einfügt, dass seine Funktion als Schloss als solche nicht für den Benutzer des Analysegerätes (1) erkennbar ist.

11. Analysegerät (1) nach einem der vorhergehenden Ansprüche, **dadurch gekennzeichnet, dass** es ein Analysegerät (1) für die Analyse einer Körperflüssigkeit, insbesondere für die Patienten-Selbstkontrolle, beispielsweise ein Blutglukosemeter, ein Cholesterinmessgerät oder ein Blutgerinnungsparameter-Messgerät ist.

12. Analysegerät (1) nach einem der vorhergehenden Ansprüche, **dadurch gekennzeichnet, dass** es ein tragbares, von einem Patienten manuell handhabbar, manuell betätigbares und manuell bedienbares Analysegerät (1) ist.

13. Verfahren zum Herstellen eines Analysegerätes (1) zum Analysieren eines medizinisch bedeutsamen Bestandteils einer Probe, insbesondere eines Blutglukosemessgerätes, wobei das Analysegerät (1) ein Gerätegehäuse (2) mit mindestens zwei Gehäuseteilen (9, 10), eine in dem Gerätegehäuse (2) angeordnete Messeinrichtung zum Durchführen der Analyse an einer Probe, und
einen Prozessor mit Software zum Verarbeiten der von der Messeinrichtung ermittelten Messwerte und zum Aufbereiten von Analysemessdaten aus den Messwerten umfasst,
und wobei das Analysegerät (1) bei seiner Herstellung einer Funktionskontrolle unterzogen und mit einem Schlosselement geschlossen wird,
**dadurch gekennzeichnet, dass**
das Analysegerät (1) mit einem Schlosselement (11) geschlossen wird, das
funktional der Verriegelung des Analysegerätes (1) durch Herstellung einer festen mechanischen Verbindung zwischen den mindestens zwei Gehäuseteilen (8, 9) dient, wobei das Analysegerät (1) und das Schlosselement (11) derart ausgebildet sind, dass das Analysegerät (1) auch ohne das Schlosselement (11) funktionsfähig ist,
derart gestaltet ist, dass bei der Montage des Analysegerätes (1) zur Durchführung einer funktionalen Endkontrolle des, bis auf das Schlosselement (11) fertig montierten Analysegerätes (1), die Funktion des Analysegerätes (1) geprüft und erforderlichenfalls das Gehäuse (2) zur Instandsetzung zerstörungsfrei geöffnet werden kann, und
derart gestaltet ist, dass bei der der Montage des Schlosselements (11) an dem Gehäuse (2), insbesondere nach Durchführung einer funktionalen Endkontrolle des Analysegerätes (1), das Gehäuse (2) verriegelt wird, so dass danach ein Wiederöffnen des Gehäuses (2) nicht mehr möglich ist, ohne dass dieses Wiederöffnen an dem Gehäuse (2) oder dem Schlosselement (11) erkennbar ist,
und dass das Schlosselement (11) als zusätzliches Gehäuseteil ausgebildet ist, das mindestens zwei Gehäuseteile (9, 10) verbindet,
und dass bei der Montage des Analysegerätes (1) zur Durchführung einer funktionalen Endkontrolle des, bis auf das Schlosselement (11) fertig montierten Analysegerätes (1), die Funktion des Analysegerätes (1) geprüft und danach bei der der Montage des Schlosselements (11) an dem Gehäuse (2), nach Durchführung einer funktionalen Endkontrolle des Analysegerätes (1), das Gehäuse (2) verriegelt wird.

14. Verfahren nach dem vorhergehenden Anspruch, **dadurch gekennzeichnet, dass** bei der Montage des Analysegerätes (1) nach Durchführung einer funktionalen Endkontrolle des, bis auf das Schlosselement (11) fertig montierten Analysegerätes (1), die Funktion des Analysegerätes (1) geprüft, im Falle eines festgestellten Funktionsfehlers das Gehäuse (2) zur Fehlerbehebung oder Instandsetzung zerstörungsfrei geöffnet und der Fehler behoben wird, anschließend zur Durchführung einer funktionalen Endkontrolle des, bis auf das Schlosselement (11) fertig montierten Analysegerätes (1), die Funktion des Analysegerätes (1) erneut geprüft und danach bei der der Montage des Schlosselements (11) an dem Gehäuse (2), nach Durchführung der erneuten funktionalen Endkontrolle des Analysegerätes (1), das Gehäuse (2) verriegelt wird.

## Claims

1. An analysis device (1) for analyzing a medically significant component of a sample, in particular a blood glucose measuring device, comprising
a device housing (2) having at least two housing parts (9, 10),
a measuring unit, situated in the device housing (2), for performing the analysis by a sample, and
a processor having software for processing the measured values ascertained by the measuring unit and for preparing analysis measurement data from the measured values,
**characterized in that**
the analysis device (1) comprises a lock element (11), which
is functionally used for locking the analysis device (1) by producing a fixed mechanical connection between the at least two housing parts (8, 9), the analysis device (1) and the lock element (11) being implemented in such a way that the analysis device (1) is functional even without the lock element (11),
is designed in such a way that upon the assembly of the analysis device (1) to perform a final function check of the analysis device (1), which is completely assembled except for the lock element (11), the function of the analysis device (1) may be tested and if necessary the housing (2) may be opened for repair without destruction, and
is designed in such a way that upon the mounting of the lock element (11) on the housing (2), in particular after performing a final function check of the analysis device (1), the housing (2) is locked, so that thereafter reopening of the housing (2) is no longer possible without this reopening being recognizable on the housing (2) or the lock element (11) and that the lock element (11) is implemented as an additional housing part which connects at least two housing parts (9, 10).

2. The analysis device (1) according to claim 1, **characterized in that** the locking of the housing (2) by the lock element (11) is implemented in such a way that damage occurs to the lock element (11) upon opening of the housing (2), by which the recognisability of the opening of the housing (2) is ensured.

3. The analysis device (1) according to any one of the preceding claims, **characterized in that** the lock element (11) engages in housing parts (9, 10) in such a way that is no longer possible to remove the lock element (11) without destruction.

4. The analysis device (1) according to any one of the preceding claims, **characterized in that** the lock element (11) has catch hooks (12), which are implemented for insertion into corresponding receptacle openings (13) of the housing (2).

5. The analysis device (1) according to any one of the preceding claims, **characterized in that** the lock element (11) has a predetermined breaking point, which breaks upon opening of the analysis device (1) and/or removal of the lock element (11).

6. The analysis device (1) according to any one of the preceding claims, **characterized in that** the lock element (11) has no predetermined breaking point, so that it is destroyed upon opening of the analysis device (1) and/or removal of the lock element (11).

7. The analysis device (1) according to any one of the preceding claims, **characterized in that** the lock element (11) is a component of the overall housing.

8. The analysis device (1) according to any one of the preceding claims, **characterized in that** the lock element (11) is implemented as a housing part or is integrated in a housing part.

9. The analysis device (1) according to any one of the preceding claims, **characterized in that** the lock element (11) is a load-bearing component of the housing construction, so that it contributes to keeping the housing parts (9, 10) mechanically together.

10. The analysis device (1) according to any one of the preceding claims, **characterized in that** the lock element (11) is included into the design of the housing (2) in such a way that its function as a lock is not recognizable as such to the user of the analysis device (1).

11. The analysis device (1) according to any one of the preceding claims, **characterized in that** it is an analysis device (1) for analyzing a bodily fluid, in particular for patient self-testing, such as a blood glucose meter, a cholesterol measuring device, or a blood coagulation parameter measuring device.

12. The analysis device (1) according to any one of the preceding claims, **characterized in that** it is a portable analysis device (1), which may be manually handled, manually actuated, and manually operated by a patient.

13. A method for producing an analysis device (1) for analyzing a medically significant component of a sample, in particular a blood glucose measuring device, the analysis device (1) comprising
a device housing (2) having at least two housing parts (9, 10),
a measuring unit, situated in the device housing (2), for performing the analysis by a sample, and
a processor having software for processing the measured values ascertained by the measuring unit and for preparing analysis measurement data from the measured values,
and the analysis device (1) being subjected to a function check and being closed using a lock element during its production,
**characterized in that**
the analysis device (1) is locked using a lock element (11), which
is functionally used for locking the analysis device (1) by producing a fixed mechanical connection between the at least two housing parts (8, 9), the analysis device (1) and the lock element (11) being implemented in such a way that the analysis device (1) is functional even without the lock element (11),
is designed in such a way that upon the assembly of the analysis device (1) to perform a final function check of the analysis device (1), which is completely assembled except for the lock element (11), the function of the analysis device (1) may be checked and if necessary the housing (2) may be opened for repair without destruction, and
is designed in such a way that upon the fitting of the lock element (11) on the housing (2), in particular after performing a final function check of the analysis device (1), the housing (2) is locked, so that thereafter reopening of the housing (2) is no longer possible without this reopening being recognizable on the housing (2) or the lock element (11),
and that the lock element (11) is implemented as an additional housing part which connects at least two housing parts (9, 10)
and upon the assembly of the analysis device (1) for performing a final function check of the analysis device (1), which is completely assembled except for the lock element (11), the function of the analysis device (1) is tested and thereafter, upon the mounting of the lock element (11) on the housing (2), after performing a final function check of the analysis device (1), the housing (2) is locked.

14. The method according to the preceding claim, **characterized in that**, upon the assembly of the analysis device (1) after performing a final function check of the analysis device (1), which is completely assembled except for the lock element (11), the function of the analysis device (1) is tested, in case of an established functional error, the housing (2) is opened without destruction for correcting the error or repair and the error is corrected, subsequently, to perform a final function check of the analysis device (1), which is completely assembled except for the lock element (11), the function of the analysis device (1) is tested again and thereafter, upon the mounting of the lock element (11) on the housing (2), after performing the final function check of the analysis device (1) again, the housing (2) is locked.

## Revendications

1. Appareil d'analyse (1) pour analyser un composant important au plan médical d'un échantillon, en particulier un appareil de mesure de glucose du sang comprenant
un boîtier d'appareil (2) avec au moins deux parties de boîtier (9, 10),
un dispositif de mesure disposé dans le boîtier d'appareil (2) pour la réalisation de l'analyse sur un échantillon, et
un processeur avec logiciel pour le traitement des valeurs mesurées déterminées par le dispositif de mesure et pour la préparation de données de mesure d'analyse à partir des valeurs mesurées,
**caractérisé en ce que**
l'appareil d'analyse (1) comporte un élément de serrure (11), qui
sert au plan fonctionnel au verrouillage de l'appareil d'analyse (1) par l'établissement d'une liaison mécanique fixe entre les au moins deux parties de boîtier (8, 9), l'appareil d'analyse (1) et l'élément de serrure (11) étant conçus de telle sorte que l'appareil d'analyse (1) est fonctionnel même sans l'élément de serrure (11),
est conçu de telle sorte que, lors du montage de l'appareil d'analyse (1) pour la réalisation d'un contrôle final fonctionnel de l'appareil d'analyse (1) monté achevé à l'exception de l'élément de serrure (11), le fonctionnement de l'appareil d'analyse (1) est testé et, si nécessaire, le boîtier (2) peut être ouvert sans destruction pour la remise en état, et
est conçu de telle sorte que, lors du montage de l'élément de serrure (11) sur le boîtier (2), en particulier après l'exécution d'un contrôle final fonctionnel de l'appareil d'analyse (1), le boîtier (2) est verrouillé de telle sorte qu'une réouverture du boîtier (2) n'est plus possible ensuite sans que cette réouverture soit perceptible sur le boîtier (2) ou l'élément de serrure (11),
et de telle sorte que l'élément de serrure (11) est conçu sous forme de partie de boîtier supplémentaire qui relie au moins deux parties de boîtier (9, 10).

2. Appareil d'analyse (1) selon la revendication 1, **caractérisé en ce que** le verrouillage du boîtier (2) par l'élément de serrure (11) est conçu de telle sorte que, lors de l'ouverture du boîtier (2), on arrive à un endommagement de l'élément de serrure (11) par laquelle la détectabilité de l'ouverture du boîtier (2) est garantie.

3. Appareil d'analyse (1) selon l'une quelconque des revendications précédentes, **caractérisé en ce que** l'élément de serrure (11) s'enclenche dans des parties de boîtier (9, 10) de telle sorte qu'un démontage sans destruction de l'élément de serrure (11) n'est plus possible.

4. Appareil d'analyse (1) selon l'une quelconque des revendications précédentes, **caractérisé en ce que** l'élément de serrure (11) présente des crochets d'encliquetage (12) qui sont conçus pour l'introduction dans des ouvertures de logement (13) correspondantes du boîtier (2).

5. Appareil d'analyse (1) selon l'une quelconque des revendications précédentes, **caractérisé en ce que** l'élément de serrure (11) présente un point de rupture qui rompt lors de l'ouverture de l'appareil d'analyse (1) ou lors de l'enlèvement de l'élément de serrure (11).

6. Appareil d'analyse (1) selon l'une quelconque des revendications précédentes, **caractérisé en ce que** l'élément de serrure (11) ne présente pas de point de rupture, de sorte qu'il est détruit lors de l'ouverture de l'appareil d'analyse (1) ou de l'enlèvement de l'élément de serrure (11).

7. Appareil d'analyse (1) selon l'une quelconque des revendications précédentes, **caractérisé en ce que** l'élément de serrure (11) est un composant du boîtier global.

8. Appareil d'analyse (1) selon l'une quelconque des revendications précédentes, **caractérisé en ce que** l'élément de serrure (11) est conçu comme partie de boîtier ou est intégré dans une partie de boîtier.

9. Appareil d'analyse (1) selon l'une quelconque des revendications précédentes, **caractérisé en ce que** l'élément de serrure (11) est une partie portante de la construction de boîtier, de telle sorte qu'il contribue à la cohérence mécanique des parties de boîtier (9, 10).

10. Appareil d'analyse (1) selon l'une quelconque des revendications précédentes, **caractérisé en ce que** l'élément de serrure (11) s'insère dans le design du boîtier (2) de telle sorte que sa fonction de serrure n'est pas perceptible en tant que telle pour l'utilisateur de l'appareil d'analyse (1).

11. Appareil d'analyse (1) selon l'une quelconque des revendications précédentes, **caractérisé en ce qu'**il s'agit d'un appareil d'analyse (1) pour l'analyse d'un liquide corporel, en particulier pour l'autocontrôle du patient, par exemple un appareil de mesure de glucose du sang, un appareil de mesure de cholestérol ou un appareil de mesure de paramètre de coagulation du sang.

12. Appareil d'analyse (1) selon l'une quelconque des revendications précédentes, **caractérisé en ce qu'**il s'agit d'un appareil d'analyse (1) portable, pouvant être manipulé manuellement par un patient, actionnable manuellement et utilisable manuellement.

13. Procédé pour fabriquer un appareil d'analyse (1) pour l'analyse d'un composant important au plan médical d'un échantillon, en particulier d'un appareil de mesure de glucose du sang, l'appareil d'analyse (1) comprenant
Un boîtier d'appareil (2) avec au moins deux parties de boîtier (9, 10),
un dispositif de mesure disposé dans le boîtier d'appareil (2) pour la réalisation de l'analyse sur un échantillon, et
un processeur avec logiciel pour le traitement des valeurs mesurées déterminées par l'appareil de mesure et pour la préparation de données de mesure d'analyse à partir des valeurs mesurées,
et l'appareil d'analyse (1) étant soumis lors de sa fabrication à un contrôle de fonctionnement et étant fermé avec un élément de serrure,
**caractérisé en ce que**
l'appareil d'analyse (1) est fermé avec un élément de serrure (11), qui sert au plan fonctionnel au verrouillage de l'appareil d'analyse (1) par l'établissement d'une liaison mécanique fixe entre les au moins deux parties de boîtier (8, 9), l'appareil d'analyse (1) et l'élément de serrure (11) étant conçus de telle sorte que l'appareil d'analyse (1) est fonctionnel même sans l'élément de serrure (11),
est conçu de telle sorte que, lors du montage de l'appareil d'analyse (1) pour la réalisation d'un contrôle final fonctionnel de l'appareil d'analyse (1) monté achevé à l'exception de l'élément de serrure (11), le fonctionnement de l'appareil d'analyse (1) est testé et, si nécessaire, le boîtier (2) peut être ouvert sans destruction pour la remise en état, et
est conçu de telle sorte que, lors du montage de l'élément de serrure (11) sur le boîtier (2), en particulier après la réalisation d'un contrôle final fonctionnel de l'appareil d'analyse (1), le boîtier (2) est verrouillé, de sorte qu'une réouverture du boîtier (2) n'est plus possible ensuite sans que cette réouverture soit perceptible sur le boîtier (2) ou l'élément de serrure (11),
et que l'élément de serrure (11) est conçu sous forme de partie de boîtier supplémentaire, qui relie au moins deux parties de boîtier (9, 10),
et de telle sorte que, lors du montage de l'appareil d'analyse (1) pour la réalisation d'un contrôle final fonctionnel de l'appareil d'analyse (1) monté achevé à l'exception de l'élément de serrure (11), le fonctionnement de l'appareil d'analyse (1) est testé et le boîtier (2) est verrouillé ensuite lors du montage de l'élément de serrure (11) sur le boîtier (2), après la réalisation d'un contrôle final fonctionnel de l'appareil d'analyse (1).

14. Procédé selon la revendication précédente, **caractérisé en ce que** lors du montage de l'appareil d'analyse (1) après la réalisation d'un contrôle final fonctionnel de l'appareil d'analyse (1) monté achevé à l'exception de l'élément de serrure (11), le fonctionnement de l'appareil d'analyse (1) est testé, le boîtier (2) est ouvert sans destruction dans le cas d'un dysfonctionnement constaté pour l'élimination du défaut ou la remise en état et le défaut est supprimé, le fonctionnement de l'appareil d'analyse (1) est testé à nouveau ensuite pour la réalisation d'un contrôle final fonctionnel de l'appareil d'analyse (1) monté achevé à l'exception de l'élément de serrure (11) et le boîtier (2) est verrouillé ensuite lors du montage de l'élément de serrure (11) sur le boîtier (2), après la réalisation du nouveau contrôle final fonctionnel de l'appareil d'analyse (1).
